# EUROPEAN PATENT APPLICATION

(11) **EP 4 755 444 A1**
(43) Date of publication of application: **10.06.2026**
(21) Application number: 24851734.4
(22) Date of filing: 01.08.2024
(51) Int. Cl.: A61N 2/02

(54) **COIL HOLDING UNIT AND TRANSCRANIAL MAGNETIC STIMULATION DEVICE**

(30) Priority: 04.08.2023 JP 2023127648
(71) Applicant: Teijin Pharma Limited, Tokyo 100-0013 (JP)
(72) Inventor: FUJIMOTO, Katsushi, Tokyo 100-0013 (JP)
(74) Representative: Hoffmann Eitle
(86) International application number: PCT/JP2024/027499
(87) International publication number: WO 2025/033309

(57) **Abstract**

[Problem]

The present invention provides a treatment coil unit enabling an rTMS treatment coil to be accurately and reliably mounted to a treatment site, and a transcranial magnetic stimulation device equipped with the same.

[Solution]

A coil holding unit for mounting a coil device for transcranial magnetic stimulation, which is equipped with a frame member having a window portion enabling observation of a magnetic stimulation site therethrough, and an indicator showing the center position of the magnetic field on mounting the coil device.

## Description

### [Technical Field]

The present invention relates to magnetic stimulation, and specifically to a treatment coil holding unit for a magnetic stimulation device, wherein the unit enables a therapist to easily align and fix a treatment coil used in a magnetic stimulation therapy device to a treatment site on the head.

### [Background Art]

In recent years, there has been growing interest in transcranial magnetic stimulation therapy as a treatment for intractable neuropathic pain, Parkinson's disease, Parkinson's syndrome, psychiatric disorders such as depression and PTSD, neurological disorders such as rehabilitation after stroke, for which drug treatment is not necessarily effective. Repetitive transcranial magnetic stimulation (rTMS) has been shown to be effective for patients unresponsive to treatment with various antidepressants, such as tricyclics, MAOIs, and SARIs, and was approved for insurance coverage in Japan in 2019 and is expected as a new treatment.

rTMS is a treatment that non-invasively alters cortical and subcortical activity by inducing eddy currents using a pulsed magnetic field to stimulate cortical neurons. That is, the eddy currents generated in the cortex stimulate neurons, causing the release of neurotransmitters, and repeated regular stimulation improves depressive symptoms.

Depression is treated by applying high frequency rTMS to the left dorsolateral prefrontal cortex of the brain. Although rTMS treatment has a low incidence of serious side effects such as seizures, side effects such as scalp pain, headaches, and discomfort are found during the treatment. The occurrence of discomfort depends on whether the coil is accurately and reproducibly placed in the right position on the patient's head. In the rTMS treatment, after a doctor and/or technician determines the required magnetic field strength and the precise location of the stimulation coil on the patient's head, subsequent treatments require the coil to be placed correctly at the determined location.

### [Citation List]

### [Patent Literature]

[PTL 1] WO 2004/080526 (JP-T-2006-520671)
[PTL 2] WO 2007/123147

### [Summary of Invention]

### [Technical Problem]

Transcranial magnetic stimulation is used for non-invasive treatment of psychiatric and neurological disorders. The treatment is effectively performed by aligning a coil center position (the center of the generated magnetic field) with the treatment position and thus minimizes influence on non-treatment sites. Furthermore, the treatment coil is required to be as close as possible to the treatment site for maximizing the therapeutic effect of the magnetic stimulation. This is because the electric field attenuates as the treatment coil moves away from the treatment site.

PTL 1 discloses a device for positioning a treatment coil in a predetermined position, wherein the device is equipped with a mounting frame member with a window portion for mounting the coil and an adjustment structure for positioning the coil at a desired position for each patient.

Such a device is equipped with a resin fixing means molded to fit the outer shape of the patient's head for positioning of each patient, and thus it takes time before the start of treatment because the frame to be mounted needs to be adjusted and formed for each patient. In addition, nose bridge is used as the origin for positioning, which has a problem of easily causing errors in the treatment position.

PTL 2 discloses a head fixture for transcranial magnetic stimulation therapy equipped with a resin shell molded to fit the shape of the subject's head. Such a fixture takes time before the start of treatment because the tool needs a custom-made shell to fit the shape of the subject's nasal root portion and ears. Furthermore, such a fixture adopts a relative positioning method of using the nose bridge as the origin for positioning, and thus is prone to errors in the treatment position.

As shown above, the transcranial magnetic therapy using conventional treatment coils and treatment coil holders uses a holder that is tailored to the shape of the subject's head, such as the shape of the nose bridge and the ears, for the subject to adjust the coil installation position so as to align easily the treatment position to the coil center. For this reason, the above therapy adopts a method of estimating the treatment position from an indirect positional relationship based on the subject's head shape, and thus makes it difficult to guarantee exact positioning to the treatment position point which is dependent on the individual patient's head shape and head size, and thus to minimize the positional misalignment between the treatment position and the treatment coil. Although the above problem could be solved by providing a dedicated holder for each patient, this would require a custom-made holder for each patient, causing a problem of increased costs.

In addition, use of publicly known technologies of cameras and 3D positioning systems enables guarantee of accurate positioning, but necessitates a high-cost device configuration requiring devices and systems for positioning, etc., which becomes a barrier to introduction of the device.

### [Solution to Problem]

The present invention provides a treatment coil unit, and a transcranial magnetic stimulation device equipped with the same, as described below, which is a device capable of mounting accurately and reliably an rTMS treatment coil to a treatment site.
1) A coil holding unit holding at a predetermined position a coil device generating a magnetic field for applying magnetic stimulation to a user, comprising: a coil mounting portion for mounting and fixing a coil device; a frame member having a window portion enabling observation of a magnetic stimulation site therethrough; and an indicator for showing the center position of a magnetic field on mounting a coil device to a window portion of the frame member; wherein magnetic stimulation is applied to a predetermined position by aligning the position of the indicator with a treatment site marked on a user's skin.
2) The coil holding unit according to 1), wherein the indicator is a fibrous or tape-like member stretched across a frame member constituting the window portion, and at least comes into contact with a treatment site of a head on mounting a coil device to a coil holding unit.
3) The coil holding unit according to 1), wherein the frame member and an indicator are non-magnetic members.
4) The coil holding unit according to 1), wherein the frame member covers a user's head and is equipped with a window portion enabling observation therethrough an area including at least a dorsolateral prefrontal cortex of a user's head.
5) The coil holding unit according to 1), further comprising a fixing means for fixing the coil holding unit to a user's jaw and occiput in an aligned condition of the indicator and a treatment site at a predetermined position.
6) The coil holding unit according to 1), further comprising at least two protruding portions for mounting a coil device at a predetermined position.
7) The coil holding unit according to 1), wherein the frame member covers a user's head, and is equipped with a guide bar mounted on the frame member at a user's front side along a user's midline and defines the position of a frame member to the tip of a patient's nose.
8) The coil holding unit according to 7), wherein the end of the guide bar is equipped with an indicator portion for indicating the position of the tip of a user's nose.
9) A transcranial magnetic stimulation device comprising: a coil device generating a magnetic field for applying magnetic stimulation to a user's head, and a coil holding unit holding the coil device at a predetermined position on a head, wherein the coil holding unit is equipped with a coil mounting portion for mounting a coil device to a head; a frame member equipped with a window portion enabling observation therethrough a magnetic stimulation site; an indicator for showing the center position of a magnetic field on mounting a coil device to the window portion; and a fixture for fixing the coil holding unit with the position of the indicator aligned to a marked treatment site.
10) The transcranial magnetic stimulation device according to 9), wherein the frame member covers a user's head, and is equipped with a guide bar mounted on the frame member at a user's front side along a user's midline and defines the position of a frame member to the tip of a patient's nose.

### [Advantageous Effects of Invention]

When placing a treatment coil of a transcranial magnetic stimulation device to a treatment site of a patient who is a user at the same time, it is difficult to confirm whether the center of the magnetic field of the treatment coil is accurately aligned with the treatment site. The transcranial magnetic stimulation device of the present invention is equipped with a treatment coil holding unit that fixes the treatment coil so that the treatment position on the patient's head coincides with the center of the magnetic field of the treatment coil. Visual alignment of the indicator, equipped in the treatment coil holding unit, with the treatment site on the patient's head enables accurate positioning of the center of the magnetic field of the treatment coil at the treatment site. Furthermore, the treatment coil can be easily and flexibly positioned and fixed to different treatment sites corresponding to various diseases, and prevent applying magnetic stimulation to the wrong site during treatment.

In addition, equipping with a guide bar on the front side of the frame member of the coil holding unit along the patient's midline to define the position to the tip of the patient's nose enables the patient by oneself to align the coil holding unit by looking in a mirror instead of being aligned at a medical institution, etc., and thus to pave the way for transcranial magnetic therapy at home.

### [Brief Description of Drawings]

[Fig. 1] A schematic diagram of the conventional transcranial magnetic stimulation device
[Fig. 2] An external view of the first embodiment of the coil holding unit of the present invention
[Fig. 3] An external view of the coil device
[Fig. 4] A schematic diagram showing a mounting method of a transcranial magnetic stimulation device equipped with the coil holding unit and the coil device of the present invention
[Fig. 5] An external view of the second embodiment of the coil holding unit of the present invention
[Fig. 6] An external view of the third embodiment of the coil holding unit of the present invention
[Fig. 7] An external view showing a mounted state in the third embodiment of the coil holding unit of the present invention
[Fig. 8] An external view showing an incorrect mounted state in the third embodiment of the coil holding unit of the present invention

### [Description of Embodiments]

The transcranial magnetic stimulation device and the treatment coil holding unit of the present invention will be described below taking an example of a treatment device for patients exhibiting depressive symptoms.

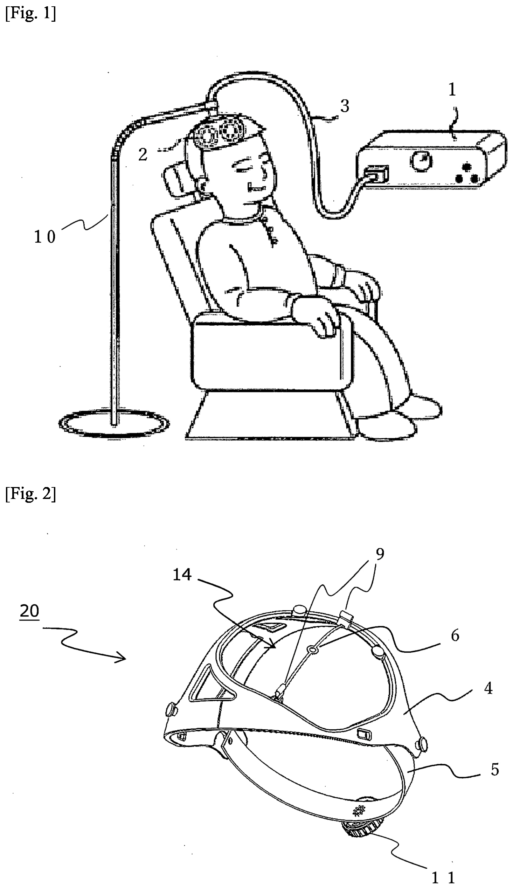

As shown in Fig. 1, the transcranial magnetic stimulation device generates a pulsed magnetic field in the coil device 2 by supplying a pulsed current from a coil drive power supply 1 via the cable 3, and can induce locally weak eddy currents by varying this magnetic field according to Faraday's law to stimulate neurons in the brain. The stimulation site on the head needs to be determined accurately, and magnetic treatment is continued while the coil device 2 is fixed to a treatment coil fixing base 10 so that the position does not shift during treatment and the patient is seated in a chair.

Various parts of the brain control emotions such as joy, anger, sadness, and happiness, as well as sleep and appetite. Patients with depressive symptoms have reduced activity in the dorsolateral prefrontal cortex (DLPFC) on the left side of the brain, and an overactive response in amygdala. rTMS activates the DLPFC through magnetic stimulation, allowing motivation and thinking to function normally and secondarily suppressing the over-response of the amygdala.

Performing rTMS treatment necessitates finding an appropriate stimulation site and intensity. The location of DLPFC is the F3 point of electroencephalogram measurement, and the stimulation site is determined based on the BeamF3 method, and then an appropriate stimulation intensity is determined. The BeamF3 method measures numerical values of three sites on the head and identifies the position of F3 based on a predetermined algorithm. F3 point is determined at the start of treatment and marked, and in the subsequent treatments, prior positioning is not required, and magnetic radiation is applied to the marking position.

It is preferable to mark directly on the head with indelible paint such as permanent marker, ink, etc. In contrast, direct marking on the head has disadvantages of making the marking position less-visible due to hair etc. and the marking fading gradually from washing the scalp in the shower etc.

Therefore, the treatment can be performed by wearing a cap like a swimming cap to fit the head and marking on the cap. Such a cap, when using mesh-like or stretchy cloth such as a swimming cap, may shift the marking position each time mounting the cap, and thus less stretchy cloth such as cotton is preferably used.

The patient wears the caps during treatment and takes off after completion of treatment, and thus needs to wear the cap in the same manner every time. The cap mounted during rTMS treatment has a line corresponding to the midline in the center of the cap. The user can wear the cap at the same position every time by aligning this line with the nose line on the face, corresponding to the midline of the user, and aligning the bottom end of the cap horizontally with the user's eyebrow line etc.

### Coil Holding Unit

Fig. 2 shows an external view of the first embodiment of the coil holding unit of the present invention.

The coil holding unit 20 is for holding a coil device, generating a magnetic field to apply magnetic stimulation to the patient's head, at a predetermined position on the head, and mounts the coil device and applies magnetic stimulation directly to the head. A coil fixing cap 4, equipped with a frame member having a window portion 14 enabling observation therethrough a magnetic stimulation site, is worn on the head and fixed to the head by a fastening band 5 of a fixture. The fastening band 5 may adopt a dial-type ratchet band 11 etc., to enable the size change of the coil holding unit corresponding to the size of the user's head and the adjustment of degree of tightening. A fixing means such as a chin strap for fixing to the patient's chin may be further provided as necessary to reduce shift of the coil during treatment.

Such window portion 14 is equipped with a treatment position indicator 6 of an indicator showing the center position of the magnetic field on mounting the coil device, wherein magnetic stimulation can be applied to a predetermined position by visually aligning the position of the treatment position indicator 6 with the marked treatment site. The treatment position indicator 6 is a fibrous or tape-like member stretched across the frame member constituting window portion 14, and equipped in the center with a ring portion for indicating the coil center position. However, the treatment position indicator 6 does not need to have a specific shape as long as the coil center position can be indicated by a shape other than a ring or by changing the color. The indicator preferably use a material such as elastomer allowing the surface of the coil device to be pressed to the head to come into contact with the head together with the treatment position indicator 6, for preventing generation of a space between the coil device and the head on mounting the coil device, and thus attenuation of the magnetic field or shifting of the magnetic field center position.

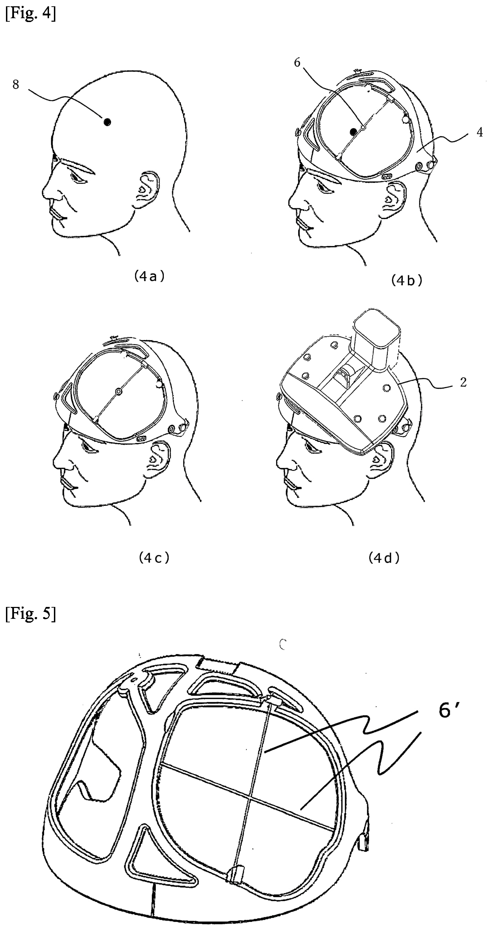

Fig. 5 shows, as the second embodiment of the treatment position indicator, a coil holding unit equipped with two tape-shaped members 6' crossing with each other. Alignment can be performed with the treatment position using the intersection of the two tapes as the coil center position.

The coil holding unit, particularly the frame members and treatment position indicators, is made from non-magnetic materials such as resin and fiber so as not to affect the magnetic field, because the coil holding unit is used for treatment by mounting a coil device to generate a magnetic field. Preferably used is a material strong enough to hold the coil device and biocompatible due to contact with the living body, specifically resin materials such as polylactic acid, polypropylene, and polyacetal. When metal parts are required for joints etc., titanium is preferably used.

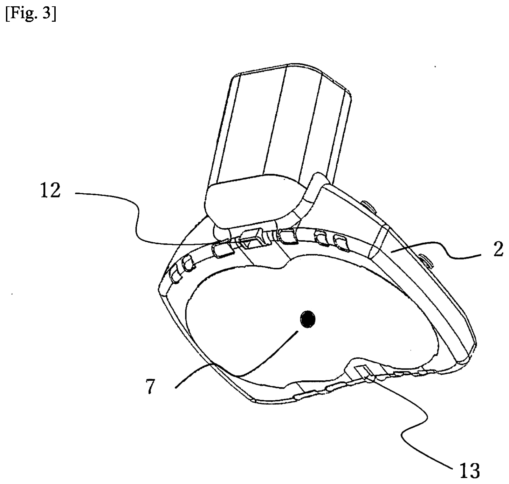

Fig. 3 shows an external view of the coil device 2. The figure shows the device viewed from the side of the scalp contact surface, and an excitation coil is inside the housing. The excitation coil is appropriately selected depending on the application, from among a normal circular coil, a figure-of-eight coil, a butterfly coil with a modified coil angle, etc. Fig. 3 shows the device with a figure-of-eight coil inside, wherein the coil center 7 indicates the center position of the magnetic field generated by the excitation coil of coil device 2, and on mounting on the coil holding unit, the position of coil center 7 coincides with the position of the ring part of treatment position indicator 6.

When the coil device 2 is mounted on the coil holding unit, the treatment position indicator 6 is pressed by the coil device 2 and comes into contact with the treatment site on the head. The treatment coil holding unit has a structure in which the magnetic field does not interfere in the area where the coil device 2 is mounted, and preferably has a structure allowing the coil device to be in close contact with the treatment site.

The coil holding unit has at least two protruding portions 9 for mounting the coil device at a predetermined position. Such protruding portions 9 fit into the concave portions 12 and 13 of the coil device and thus enable the unit to hold the device with both fixed at a predetermined position. Although such an embodiment includes the coil holding unit having protruding portions and the coil device has a fitting portion having concave portions, combination of concavity and convexity and the shape of the fitting portion can be selected appropriately.

Fig. 4 shows a mounting method of a transcranial magnetic stimulation device equipped with the coil holding unit and the coil device of the present invention.

In the magnetic therapy, a doctor locates a magnetic treatment position in the first practice and marks the treatment position on the patient's head as shown in (4a). In the case of treating depression, irradiation is applied on the DLPFC, the location of which is the F3 point of electroencephalogram measurement, and the stimulation site is determined based on the BeamF3 method. The patient wears a non-elastic cotton cap and the cap is marked.

Next, as shown in (4b), the coil holding unit of the present invention is placed over the head, and as shown in (4c), the position of the treatment position indicator 6 is aligned with the marking position, and the coil holding unit is fixed to the head. Then, as shown in (4d), the coil device is mounted to the coil holding unit, and treatment is started.

A depression treatment device needs to have the coil holding unit equipped with a window portion enabling observation of an area including the patient's dorsolateral prefrontal cortex.

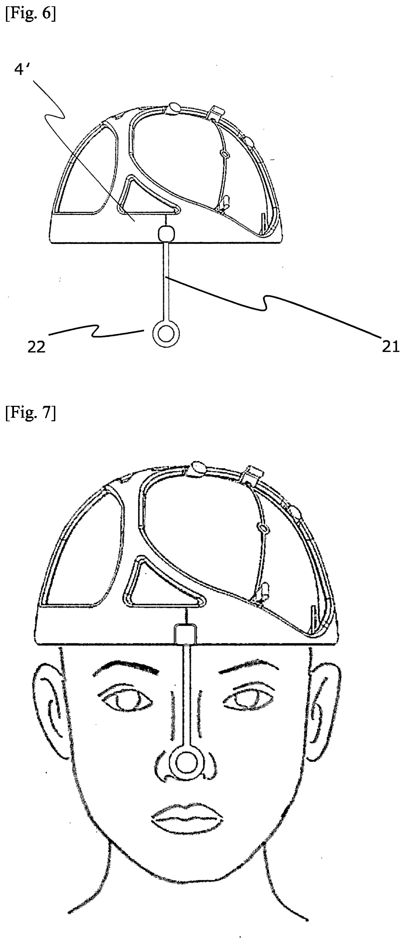

Fig. 6 shows an external view of the third embodiment of the coil holding unit of the present invention. Such an embodiment is the frame portion 4' provided with a guide bar 21 mounted along the patient's midline assuming the end of the guide bar as the tip of the nose, wherein the frame portion 4' is at the user front side of the frame portion covering the outer head circumference of the coil fixing cap 4 in the coil holding unit 20 of the first embodiment shown in Fig 2. In a medical institution etc., the position of the coil holding unit 20 is adjusted so that the treatment position 8 and the treatment position indicator 6 are aligned, and then the guide bar 21 is fixed to the frame portion 4'. There can be adopted a method of completely fixing the guide bar 21 to the frame portion 4' by adhesive or welding, and also possibly a method of indicating the fixing position.

Guide bar 21 is provided at the end with a ring-shaped indicator portion 22 indicating the tip of the user's nose. The shape of the indicator portion 22 is not particularly limited, and any shape may be used as long as it serves as a marker for the position of the tip of the nose.

As shown in Fig. 7, the user can mount the coil holding unit while looking in a mirror so that guide bar 21 is straight along the user's midline and indicator portion 22 at the end of the guide bar is positioned at the tip of the nose, wherein the position of treatment position indicator 6 of the coil holding unit is maintained to be aligned with the position of the marked treatment site, and fixing in this condition enables the user by oneself to mount the coil of the transcranial magnetic stimulation device in the correct position.

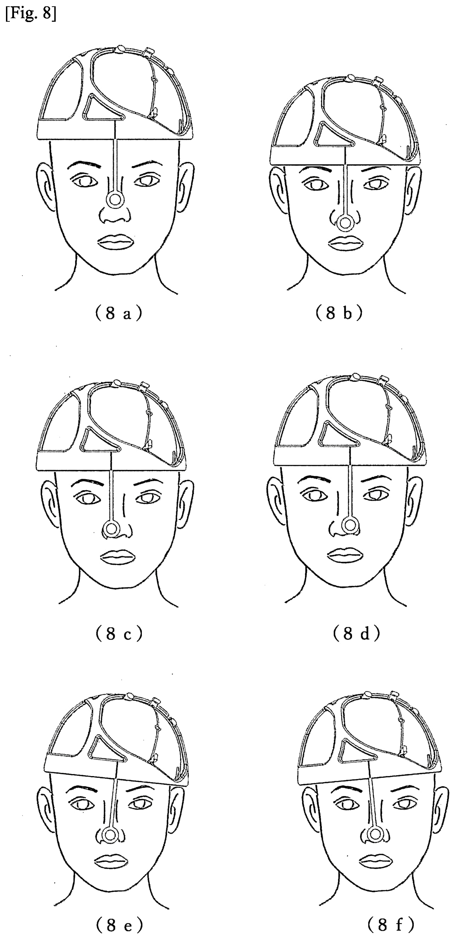

Any misalignment of the coil holding unit shown in Fig. 8, such as vertical one (8a, 8b), horizontal one (8c, 8d), or diagonal one (8e, 8f), can be checked by the user oneself while looking in a mirror, and adjusted to a correct mounting position as shown in Fig. 7, which enables treatment at home as well as treatment at a medical institution.

Such a device eliminates the need for reconfirming the treatment position, enables alignment of the transcranial magnetic stimulation device with the coil mounted beforehand to the coil holding unit, in addition to the method of mounting the coil of the transcranial magnetic stimulation device after mounting the coil holding unit.

### [Industrial Applicability]

The present invention is a coil holding unit that holds a coil device used in transcranial magnetic stimulation therapy at a predetermined position, and can be used as a transcranial magnetic stimulation device.

### [Reference Signs List]

1 Coil drive power supply
2 Coil device
3 Cable
4, 4' Coil fixing cap
5 Fastening band
6, 6' Treatment position indicator
7 Coil center
8 Treatment position
9 Protruding portion
10 Treatment coil fixing base
11 Dial-type ratchet band
12 Concave portion
13 Concave portion
14 Window portion
20 Coil holding unit
21 Guide bar
22 Indicator portion

## Claims

1. A coil holding unit holding at a predetermined position a coil device generating a magnetic field for applying magnetic stimulation to a user, comprising: a coil mounting portion for mounting and fixing a coil device; a frame member having a window portion enabling observation of a magnetic stimulation site therethrough; and an indicator for showing the center position of a magnetic field on mounting a coil device to a window portion of the frame member; wherein magnetic stimulation is applied to a predetermined position by aligning the position of the indicator with a marked treatment site.

2. The coil holding unit according to claim 1, wherein the indicator is a fibrous or tape-like member stretched across a frame member constituting the window portion, and at least comes into contact with a treatment site on mounting a coil device to a coil holding unit.

3. The coil holding unit according to claim 1, wherein the frame member and the indicator are non-magnetic members.

4. The coil holding unit according to claim 1, wherein the frame member covers a user's head and is equipped with a window portion enabling observation therethrough an area including at least a dorsolateral prefrontal cortex of a user's head.

5. The coil holding unit according to claim 1, further comprising a fixing means for fixing the coil holding unit to a user's jaw and occiput in an aligned condition of the indicator and a treatment site at a predetermined position.

6. The coil holding unit according to claim 1, wherein the coil mounting portion has at least two fitting portions for mounting and fixing a coil device to a coil holding unit.

7. The coil holding unit according to claim 1, wherein the frame member covers a user's head, and is equipped with a guide bar mounted on the frame member at a user's front side along a user's midline and defines the position of a frame member to the tip of a user's nose.

8. The coil holding unit according to claim 7, wherein the end of the guide bar is equipped with an indicator portion for indicating the position of the tip of a user's nose.

9. A transcranial magnetic stimulation device comprising: a coil device generating a magnetic field for applying magnetic stimulation to a user's head, and a coil holding unit holding the coil device at a predetermined position on a head, wherein the coil holding unit is equipped with a coil mounting portion for mounting a coil device to a head; a frame member equipped with a window portion enabling observation therethrough a magnetic stimulation site; an indicator for showing the center position of a magnetic field on mounting a coil device to the window portion; and a fixture for fixing the coil holding unit with the position of the indicator aligned to a marked treatment site.

10. The transcranial magnetic stimulation device according to claim 9, wherein the frame member covers a user's head, and is equipped with a guide bar mounted on the frame member at a user's front side along a user's midline and defines the position of a frame member to the tip of a user's nose.
